(19) Europäisches Patentamt
European Patent Office
Office européen des brevets

(11) Veröffentlichungsnummer: **0 252 515 A1**

(12) # EUROPÄISCHE PATENTANMELDUNG

(21) Anmeldenummer: **87109986.7**

(22) Anmeldetag: **10.07.87**

(51) Int. Cl.³: **C 07 D 239/50**

(30) Priorität: **10.07.86 HU 285586**

(43) Veröffentlichungstag der Anmeldung:
**13.01.88 Patentblatt 88/2**

(84) Benannte Vertragsstaaten:
**AT BE CH DE ES FR GB GR IT LI NL SE**

(71) Anmelder: **RICHTER GEDEON VEGYESZETI GYAR R.T.**
**Gyömrői ut 19-21**
**H-1475 Budapest X(HU)**

(72) Erfinder: **Vedres, András, Dr.**
**Füredi u. 56**
**H-1144 Budapest(HU)**

(72) Erfinder: **Szántay, Csaba, Dr.**
**Zsombolyai u. 8**
**H-1113 Budapest(HU)**

(72) Erfinder: **Stefkó, Béla, Dr.**
**Orlay u. 2/b**
**H-1117 Budapest(HU)**

(72) Erfinder: **Kreidl, János, Dr.**
**Pusztaszeri u. 11**
**H-1025 Budapest(HU)**

(72) Erfinder: **Nemes, András, Dr.**
**Hankóczi u. 11**
**H-1022 Budapest(HU)**

(72) Erfinder: **Blaskó, Gábor, Dr.**
**Molnár Erik u. 21**
**H-1113 Budapest(HU)**

(72) Erfinder: **Bogsch, Erik**
**Lidéric u. 40-42**
**H-1121 Budapest(HU)**

(72) Erfinder: **Máthé, Dénes**
**Eszék u. 13/15**
**H-1114 Budapest(HU)**

(72) Erfinder: **Hegedüs, István**
**Balogh A. u. 7/b**
**H-1026 Budapest(HU)**

(72) Erfinder: **Szuchovszky, Adrienn, Dr.**
**Pava u. 34**
**H-1094 Budapest(HU)**

(72) Erfinder: **Mester, Tamás**
**Borsó u. 56**
**H-1173 Budapest(HU)**

(74) Vertreter: **Beszédes, Stephan G. Dr.**
**Münchener Strasse 80a Postfach 1168**
**D-8060 Dachau(DE)**

(54) **Vefahren zur Herstellung von 6-(Amino)-1,2-di-(hydro)-1-(hydroxy)-2-(imino)-4-(piperidino)-pyrimidin.**

(57) Gegenstand der Erfindung ist ein Verfahren zur Herstellung von 6-(Amino-1,2-di-(hydro)-1-(hydroxy)-2-(imino)-4-(piperidino)-pyrimidin der Formel

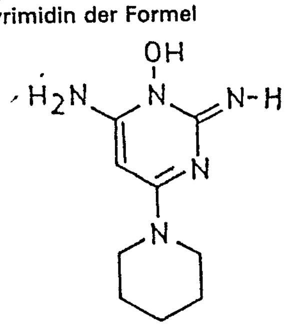

I ,

bei welchem [eine] Acyl- und/oder Acyloxygruppe(n) aufweisenden 2-(Imino)-pyrimidinderivate der allgemeinen Formel

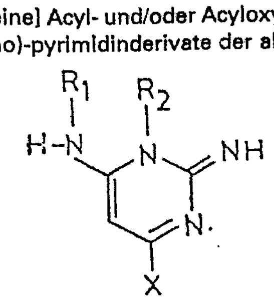

II ,

worin

$R_1$ für ein Wasserstoffatom oder einen Rest der allgemeinen Formel

./...

**EP 0 252 515 A1**

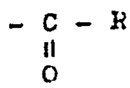

III ,

in welchletzterer

R einen Alkylrest oder einen, gegebenenfalls durch ein Halogenatom substituierten, Arylrest bedeutet,

steht.

$R_2$ eine Hydroxygruppe oder einen Rest der allgemeinen Formel

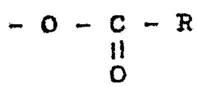

IV ,

in welchletzterer

R die obigen Bedeutungen hat,

darstellt und

X für ein Chlor- oder Bromatom oder einen, gegebenenfalls substituierten, Arylsulfonyloxyrest steht,

mit der weiteren Maßgabe, daß,

im Falle daß

$R_1$ für ein Wasserstoff atom steht,

$R_2$ von einer Hydroxygruppe verschieden ist,

mit Piperidin umgesetzt und die erhaltenen [eine] Acyl- und/oder Acyloxygruppe(n) aufweisenden 2-(Imino)-4-(piperidino)-pyrimidinderivate der allgemeinen Formel

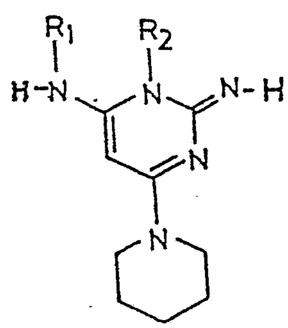

V ,

worin

$R_1$ und $R_2$ die obigen Bedeutungen haben,

hydrolysiert werden.

Das 6-(Amino)-1,2-di-(hydro)-1-(hydroxy)-2-(imino)-4-(piperidino)-pyrimidin hat eine blutdrucksenkende Wirkung.

0252515

## Beschreibung

Gegenstand der Erfindung ist ein neues Verfahren zur Herstellung von 6-(Amino)-1,2-di-(hydro)-1-(hydroxy)-2--(imino)-4-(piperidino)-pyrimidin der Formel

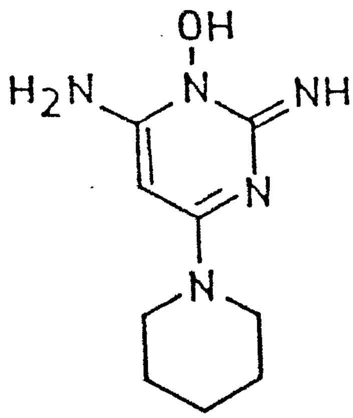

(I) .

Diese Verbindung (internationale freie Kurzbezeichnung: Minoxidil) zeichnet sich durch eine hervorragende blutdrucksenkende Wirkung aus (Drugs 22 [1981], 257) und ihr Wirkstoff ist in zahlreichen Ländern als blutdrucksenkendes Mittel bekannt. In letzter Zeit gerät die Nutzung der Verbindung als Heilkosmetikum immer mehr in den Vordergrund, da sie in verdünnter Lösung äußerlich angewandt das Haarwachstum wirksam anregt (Pharm. Ind. 46 [1984], 937 und Pharm. Ind. 47 [1985], 506). Im Fachschrifttum hat die Bezeichnung Minoxidil - da die Verbindung in 2 tautomeren Formen vorkommen kann - 2-fache Bedeutung. In den Chemical Abstracts wurde die Verbindung vor 1972 unter dem Namen 6-(Amino)-1,2-di-(hydro)--1-(hydroxy)-2-(imino)-4-(piperidino)-pyrimidin erwähnt, während sie danach unter der Bezeichnung 6-(1-Piperidinyl)--2,4-pyrimidindiamin-3-oxyd referiert wird. Im vorliegenden Anmeldungstext wird der erstere Name verwendet, da dieser den chemischen Charakter des erfindungsgemäßen Herstellungsverfahrens widerspiegelt, das erfindungsge-

- 3 -

mäße Verfahren umfaßt aber sinngemäß die Herstellung von beiden tautomeren Formen.

Für die Herstellung des 6-(Amino)-1,2-di-(hydro)-1--(hydroxy)-2-(imino)-4-(piperidino)-pyrimidines der Formel I sind mehrere Verfahren bekannt, keines dieser Verfahren kann jedoch als befriedigendes, eine wirtschaftliche technische beziehungsweise industrielle Herstellung ermöglichendes Verfahren angesehen werden, und zwar teils wegen der geringen Ausbeute, teils wegen der schlecht zugänglichen Ausgangsstoffe.

Die Synthese des 6-(Amino)-1,2-di-(hydro)-1-(hydroxy)--2-(imino)-4-(piperidino)-pyrimidines der Formel I wurde zuerst von 2,6-Di-(amino)-4-(chlor)-pyrimidin ausgehend verwirklicht (britische Patentschrift 1 167 735; C.A. 68, 21947h). Die Ausgangsverbindung wurde bei 150°C in Gegenwart von 85%-iger wäßriger Kaliumhydroxydlösung mit 2,4-Di--(chlor)-phenol erhitzt, das erhaltene 2,6-Di-(amino)-4--[2',4'-di-(chlor)-phenoxy]-pyrimidin wurde mit geringer Ausbeute mit m-(Chlor)-perbenzoesäure zu 6-[Amino]-4-[2',4'--di-(chlor)-phenoxy]-2,4-di-[hydro]-1-[hydroxy]-2-[imino]--pyrimidin oxydiert, diese letztere Verbindung wurde dann durch Erhitzen mit Piperidin auf 150°C in die gewünschte Verbindung 6-(Amino)-1,2-di-(hydro)-1-(hydroxy)-2-(imino)--4-(piperidino)-pyrimidin überführt. Die Gesamtausbeute der Synthese beträgt lediglich etwa 2,5% und auch die Ausbeute des letzten Schrittes liegt nur um etwa 45%. Der Grund hierfür liegt in erster Linie darin, daß die 2,4-Di-(chlor)--phenoxygruppe nur unter energischen Reaktionsbedingungen und in einer langen Reaktionszeit, die nicht erwünschte Nebenreaktionen begünstigen, gegen Piperidin ausgetauscht werden kann.

Ebenso kommt auch das in der Veröffentlichung J. Org. Chem. 4 [1975], 3 304 beschriebene Verfahren unter dem Gesichtspunkt der technischen beziehungsweise industriellen Durchführung nicht in Frage, da für den Schlüsselschritt der Synthese, für das Reaktionsfähigmachen der Carbonylgruppe des Cyanacetylpiperidin-säureamides, besondere Bedingungen (Ausschließen von Feuchtigkeit, sehr niedrige Temperatur) und schwer zugängliche und mit hohem Aufwand verbundene Stoffe ("magic-methyl", Trimethyl-oxonium-fluoborat) erforderlich sind.

Die weiteren Verfahren, wie das in der DE-OS 2 114 887 beschriebene Verfahren, in welchem als Ausgangsstoff das 4-(Chlor)-derivat von 6-(Amino)-1,2-di-(hydro)-1-(hydroxy)--2-(imino)-pyrimidin verwendet wird, oder das Verfahren der HU-PS 177 601, bei welchem vom 4-(p-Toluol-sulfonyloxy)--derivat dieser Verbindung ausgegangen wird, liefern das 6-(Amino)-1,2-di-(hydro)-1-(hydroxy)-2-(imino)-4-(piperidino)-pyrimidin der Formel I schon in einer etwas besseren, jedoch immer noch nicht befriedigenden Ausbeute. So kann gemäß den Beispielen der HU-PS 177 601 die Reaktion mit einer Ausbeute von 55 bis 65% durchgeführt werden. Eine weitere Verbesserung der Ausbeute wird jedoch durch die verhältnismäßig lange Reaktionszeit und die hohe Reaktionstemperatur verhindert, die auch hier die Bildung von Nebenprodukten begünstigen. Die infolge der Beschädigung der freien Aminogruppen des Moleküles entstandenen Nebenprodukte verunreinigen das Endprodukt, weshalb zur Herstellung der letzteren in entsprechender Reinheit eine weitere Reinigung erforderlich ist. Ein weiterer Nachteil dieser Verfahren besteht darin, daß die Herstellung der Ausgangsstoffe durch die Oxydation mit Perbenzoesäure oder m-Chlor-perbenzoesäure ziemlich umständlich ist.

Der Erfindung liegt daher die Aufgabe zugrunde, unter Behebung der Nachteile der bekannten Verfahren ein technisch beziehungsweise industriell und wirtschaftlich durchführbares Verfahren zur Herstellung des 6-(Amino)-1,2-di--(hydro)-1-(hydroxy)-2-(imino)-4-(piperidino)-pyrimidines der Formel I, durch welches diese Verbindung leicht, in guter Ausbeute und in großer Reinheit hergestellt werden kann, zu schaffen.

Das Obige wurde überraschenderweise durch die Erfindung erreicht.

Gegenstand der Erfindung ist ein Verfahren zur Herstellung von 6-(Amino)-1,2-di-(hydro)-1-(hydroxy)-2-(imino)-4--(piperidino)-pyrimidin der Formel

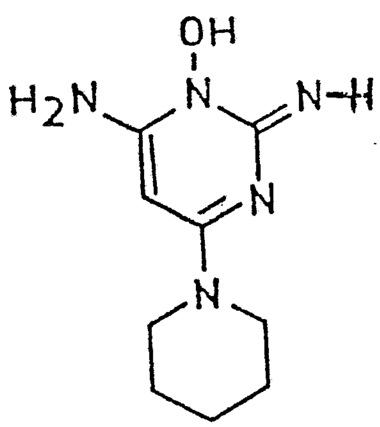

I ,

welches dadurch gekennzeichnet ist, daß [eine] Acyl- und/ /oder Acyloxygruppe(n) aufweisenden 2-(Imino)-pyrimidinderivate der allgemeinen Formel

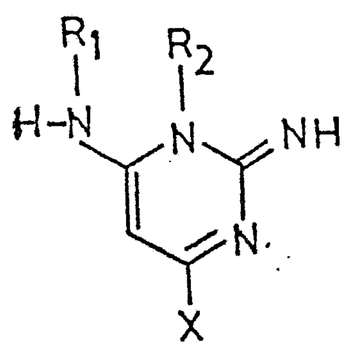

II ,

worin

$R_1$ für ein Wasserstoffatom oder einen Rest der allgemeinen Formel

$$-\underset{\underset{O}{\|}}{C}-R \qquad \text{III} \quad ,$$

in welchletzterer

R einen Alkylrest mit 1 bis 6 Kohlenstoffatom(en) oder einen, gegebenenfalls durch ein Halogenatom substituierten, Arylrest bedeutet,

steht,

$R_2$ eine Hydroxygruppe oder einen Rest der allgemeinen Formel

$$-O-\underset{\underset{O}{\|}}{C}-R \qquad \text{IV} \quad ,$$

in welchletzterer

R die obigen Bedeutungen hat,

darstellt und

X für ein Chlor- oder Bromatom oder einen, gegebenenfalls 1- oder mehrfach substituierten, Arylsulfonyloxyrest steht,

mit der weiteren Maßgabe, daß,

im Falle daß

$R_1$ für ein Wasserstoffatom steht,

$R_2$ von einer Hydroxygruppe verschieden ist,

mit Piperidin umgesetzt und die erhaltenen [eine] Acyl- und/oder Acyloxygruppe(n) aufweisenden 2-(Imino)-4--(piperidino)-pyrimidinderivate der allgemeinen Formel

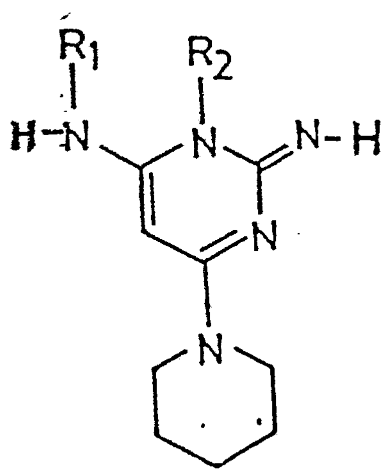

V ,

worin

$R_1$ und $R_2$ die obigen Bedeutungen haben,

gegebenenfalls nach ihrem Isolieren, hydrolysiert werden.

In den als Ausgangssubstanzen eingesetzten [eine] Acyl- und/oder Acyloxygruppe(n) aufweisenden 2-(Imino)--pyrimidinderivaten der allgemeinen Formel II kann der Alkylrest, für den R stehen kann, jeder beliebige geradkettige oder verzweigte gesättigte Kohlenwasserstoffrest mit 1 bis 6 Kohlenstoffatom(en), zum Beispiel ein Methyl-, Äthyl-, n- beziehungsweise Isopropyl-, n-, sek.- beziehungsweise tert.-Butyl-, n- beziehungsweise Isopentyl- beziehungsweise n- beziehungsweise Isohexylrest, sein. Vorzugsweise ist der Alkylrest, für den R stehen kann, ein solcher mit 1 bis 4, insbesondere 1 oder 2, ganz besonders 1, Kohlenstoffatom(en).

Es ist auch bevorzugt, daß in den als Ausgangssubstanzen eingesetzten [eine] Acyl- und/oder Acyloxygruppe(n) aufweisenden 2-(Imino)-pyrimidinderivaten der allgemeinen Formel II der Arylrest, für den R stehen kann, ein solcher mit 6 bis 12 Kohlenstoffatomen, insbesondere ein Phenyl- oder Naphthylrest, ganz besonders ein Phenylrest, ist. Vorzugsweise ist der substituierte Arylrest, für den R stehen kann, durch 1 oder 2 Substituent(en) substituiert. Ferner ist es besonders bevorzugt, daß das beziehungsweise die Halogenatom(e), durch welche[s] der Arylrest, für den R stehen kann, substituiert sein kann, [ein] Chlor- und/oder Bromatom(e), insbesondere Chloratom(e), ist beziehungsweise sind.

Weiterhin ist es bevorzugt, daß in den als Ausgangssubstanzen eingesetzten [eine] Acyl- und/oder Acyloxygruppe(n) aufweisenden 2-(Imino)-pyrimidinderivaten der allgemeinen Formel II der Arylsulfonyloxyrest, für den X stehen kann, ein solcher mit einem Arylteil mit 6 bis 12 Kohlenstoffatomen, insbesondere ein Phenylsulfonyloxy- oder Naphthylsulfonyloxyrest, ganz besonders ein Phenylsulfonyloxyrest, ist.

Vorzugsweise ist in den als Ausgangssubstanzen eingesetzten [eine] Acyl- und/oder Acyloxygruppe(n) aufweisenden 2-(Imino)-pyrimidinderivaten der allgemeinen Formel II

der substituierte Arylsulfonyloxyrest, für den X stehen kann, durch 1 bis 3 Substituent(en) substituiert. Besonders bevorzugt ist es, daß der beziehungsweise die Substituent(en) am Arylring des Arylsulfonyloxyrestes, für den X stehen kann, [ein] Alkylrest(e) mit 1 bis 4, vor allem 1 bis 3, insbesondere 1 oder 2, ganz besonders 1, Kohlenstoffatom(en) ist beziehungsweise sind. Ganz besonders bevorzugt ist der Arylsulfonyloxyrest, für den X stehen kann, ein Toluolsulfonyloxyrest (Tosyloxyrest), vor allem p-Toluolsulfonyloxyrest, oder Mesitylensulfonyloxyrest. Am meisten bevorzugt steht X für ein Chloratom.

Der Schlüsselschritt des erfindungsgemäßen Verfahrens ist die zwischen den [eine] Acyl- und/oder Acyloxygruppe(n) aufweisenden 2-(Imino)-pyrimidinderivaten der allgemeinen Formel II und dem Piperidin stattfindende nukleophile Substitution, die dank den elektronenanziehenden Eigenschaften der Acyl- beziehungsweise Acyloxyreste, für welche $R_1$ und/oder $R_2$ steht beziehungsweise stehen, unter gemäßigten Reaktionsbedingungen leicht abläuft. Diese Umsetzung der [eine] Acyl- und/oder Acyloxygruppe(n) aufweisenden 2-(Imino)-pyrimidinderivate der allgemeinen Formel II mit dem Piperidin wird zweckmäßig bei Temperaturen von 0 bis 100°C, vorzugsweise Raumtemperatur, durchgeführt. Es ist auch zweckmäßig, sie in einem Lösungsmittel vorzunehmen. Nach einer zweckmäßigen Ausführungsform des erfindungsgemäßen Verfahrens wird daher das Umsetzen der [eine] Acyl- und/oder Acyloxygruppe(n) aufweisenden 2-(Imino)--pyrimidinderivate der allgemeinen Formel II mit dem Piperidin bei Raumtemperatur und in Lösungsmitteln durchgeführt. Die Wahl der Reaktionstemperatur hängt von den übrigen Reaktionsbedingungen, wie vom Lösungsmittel, ab. Die Reaktionszeit beträgt im allgemeinen 5 Minuten bis einige Stunden. Im allgemeinen sind auch bei Raumtemperatur höchstens 2 Stunden für den Ablauf der Reaktion notwendig, während mit einer Erhöhung der Temperatur die Reaktionszeit auch im Vergleich hierzu bedeutend verkürzt werden kann. Bei der Umsetzung kann das Piperidin selbst als Lösungsmittel dienen, es können aber auch andere Lösungsmittel verwendet werden. So wird nach einer zweckmäßigen Ausführungsform des erfindungsgemäßen Verfahrens als Lösungsmittel für die Umsetzung der [eine] Acyl- und/oder Acyloxygruppe(n) aufweisenden 2-(Imino)-pyrimidinderivate der allgemeinen Formel II mit dem Piperidin das Piperidin selbst verwendet. Nach einer anderen zweckmäßigen Ausführungsform des erfindungsgemäßen Verfahrens werden als Lösungsmittel für die Umsetzung der [eine] Acyl- und/oder Acyloxygruppe(n) aufweisenden 2--(Imino)-pyrimidinderivate der allgemeinen Formel II mit dem Piperidin protische Lösungsmittel, insbesondere Äthanol, und/oder dipolare aprotische Lösungsmittel, insbesondere Acetonitril, und/oder apolare aprotische Lösungsmittel, insbesondere Chloroform, verwendet. Unter solchen Bedingungen werden die [eine] Acyl- und/oder Acyloxygruppe(n) aufweisenden 2-(Imino)-4-(piperidino)--pyrimidinderivate der allgemeinen Formel V praktisch quantitativ erhalten.

Von den erhaltenen [eine] Acyl- und/oder Acyloxygruppe(n) aufweisenden 2-(Imino)-4-(piperidino)-pyrimidinderivaten der allgemeinen Formel V kann beziehungsweise können die Acyl- und/oder Acyloxygruppe(n), wie die Acetyl- beziehungsweise Acetoxygruppe(n), für welche $R_1$ und/oder $R_2$ steht beziehungsweise stehen, überraschend leicht hydrolytisch abgespalten werden. Dieser Vorgang läuft, wenn das Piperidin auch die Aufgabe des Lösungsmittels erfüllt, bereits bei Raumtemperatur schnell ab, so daß die [eine] Acyl- und/oder Acyloxygruppe(n) aufweisenden 2-(Imino)-4-(piperidino)-pyrimidinderivate der allgemeinen Formel V nicht einmal isolierbar sind, oder er geht innerhalb Minuten unter den Bedingungen der Aufarbeitung, auf Einwirkung von Wasser und einer Base, zum

Beispiel von wäßriger Alkalilauge, vor sich. Das Verfahren kann auch so gelenkt werden, daß die Zwischenprodukte [eine] Acyl- und/oder Acyloxygruppe(n) aufweisenden 2-(Imino)-4-(piperidino)-pyrimidinderivate der allgemeinen Formel V in hoher Ausbeute und in hoher Reinheit isolierbar sind. Nach einer zweckmäßigen Ausführungsform des erfindungsgemäßen Verfahrens werden daher die [eine] Acyl- und/oder Acyloxygruppe(n) aufweisenden 2-(Imino)-4-(piperidino)-pyrimidinderivate der allgemeinen Formel V vor der Hydrolyse isoliert.

Das erfindungsgemäß hergestellte 6-(Amino)-1,2-di-(hydro)-1-(hydroxy)-2-(imino)-4-(piperidino)-pyrimidin der Formel I ist in kristalliner Form in hoher Reinheit isolierbar und wird nicht von nachweisbaren Nebenprodukten begleitet.

Beim erfindungsgemäßen Verfahren sind die Nachteile der bekannten Verfahren zur Herstellung von 6-(Amino)-1,2-di-(hydro)-1-(hydroxy)-2-(imino)-4-(piperidino)-pyrimidin der Formel I behoben.

Die wichtigsten Vorteile des erfindungsgemäßen Verfahrens sind zusammengefaßt wie folgt:

a) Die als Ausgangssubstanzen dienenden [eine] Acyl- und/oder Acyloxygruppe(n) aufweisenden 2-(Imino)-pyrimidinderivate der allgemeinen Formel II können leicht und in guter Ausbeute nach dem in der ungarischen Patentanmeldung mit dem Aktenzeichen 2856/86 und dem Prioritätstag vom 10. Juli 1986 entsprechenden gleichzeitig eingereichten europäischen Patentanmeldung derselben Anmelderin beschriebenen Verfahren hergestellt werden.

b) Diese Ausgangssubstanzen sind reaktionsfähiger als alle bekannten Ausgangsstoffe. Dies kann auf die elektronenanziehende Eigenschaft der Acyl- beziehungsweise Acyloxygruppen zurückgeführt werden, als deren Folge sich die Elektronendichte in der 4-Stellung des Pyrimidinringes verringert und die nukleophile Substitution des Piperidines leichter verläuft.

c) Die Acyl- beziehungsweise Acyloxygruppen schützen gleichzeitig die empfindlichsten Stellen des Moleküles, weshalb das Verfahren von keinerlei Nebenreaktion begleitet wird.

d) Die Acyl- beziehungsweise Acyloxygruppen sind überraschenderweise leicht entfernbar, so daß ihre Hydrolyse auf Einwirkung einer äquivalenten Menge Lauge bei Raumtemperatur innerhalb Minuten ablaufen kann. Diese leichte Entfernbarkeit ist eine strukturelle Eigenheit des Moleküles, für welche die N-(Oxy)-Struktur verantwortlich ist. Die Hydrolyse der Acylgruppe erfolgt von dem am Stickstoffatom in der 1-Stellung befindlichen Sauerstoffatom. Die an einer anderen Stelle befindliche Acylgruppe wandert auf Einwirkung von Säuren oder Laugen, aber auch auf einfaches Lösen hin schnell an diese Stelle, von wo sie mit einer den Estern entsprechenden Geschwindigkeit abhydrolysiert.

Alle diese Vorteile haben zum Ergebnis, daß mit dem erfindungsgemäßen Verfahren unter gemäßigten Reaktionsbedingungen und unter Verwendung von leicht zugänglichen und in guter Ausbeute herstellbaren Ausgangssubstanzen das Endprodukt 6-(Amino)-1,2-di-(hydro)-1-(hydroxy)-2-(imino)-4-(piperidino)-pyrimidin der Formel I in einer

hervorragend hohen und zwar unter optimalen Bedingungen 70 bis 80%-igen, Ausbeute und in hoher Reinheit hergestellt werden kann.

Die im erfindungsgemäßen Verfahren als Ausgangssubstanzen verwendeten [eine] Acyl- und/oder Acyloxygruppe(n) aufweisenden 2-(Imino)-pyrimidinderivate der allgemeinen Formel II sind neue Verbindungen, die wie bereits gesagt nach dem in der der ungarischen Patentanmeldung mit dem Aktenzeichen 2856/86 und dem Prioritätstag vom 10. Juli 1986 entsprechenden gleichzeitig eingereichten europäischen Patentanmeldung derselben Anmelderin beschriebenen Verfahren aus entsprechenden, in der 4-Stellung durch einen Substituenten X substituierten 2,6-Di-(amino)-pyrimidinderivaten in der Weise hergestellt werden können, daß diese in Gegenwart von Wasser und Wasserstoffperoxyd mit entsprechenden Säureanhydriden umgesetzt werden.

Die Erfindung wird an Hand der folgenden Beispiele näher erläutert.

<u>Beispiel 1</u>

Herstellung von 6-Amino-1,2-dihydro-1-hydroxy-2-imino--4-piperidino-pyrimidin

Zum Gemisch von 10 ml Äthanol und 3 ml Piperidin werden unter Rühren 1,01 g (5 mMol) 6-Amino-1,2-dihydro-1-acetoxy--2-imino-4-chlorpyrimidin gegeben. Das Gemisch wird unter Rühren eine halbe Stunde lang gekocht. Dann werden 5 ml wässrigen Natriumhydroxydlösung zugegeben, und es wird eine weitere halbe Stunde lang gekocht. Das Gemisch wird im Vakuum eingedampft und der Rückstand mit 10 ml Wasser vermischt. Die erhaltenen Kristalle werden abfiltriert, mit Wasser gewaschen und getrocknet.

So erhält man 0,85 g (82 %) der Titelverbindung.
Schmelzpunkt: 262 - 266 °C.
IR-Spektrum ($cm^{-1}$): 3450, 3420, 3400, 3370, 3260, 1655, 1250, 1210, 1165, 1020
$^1H$-NMR-Spektrum (DMSO-$d_6$): 1,52; 3,40; 5,36; 6,84
$^{13}C$-NMR-Spektrum (DMSO-$d_6$ + $CD_3OD$): 156,6; 153,7; 152,1; 74,1; 45,7; 25,7; 24,7.

Beispiel 2

Herstellung von 6-Amino-1,2-dihydro-1-hydroxy-2-imino--4-piperidino-pyrimidin

Unter Rühren werden 2,02 g (10 mMol) 6-Amino-1,2-dihydro--1-acetoxy-2-imino-4-chlorpyrimidin bei Raumtemperatur zu 8 ml Piperidin gegeben. Das Gemisch wird bei Raumtemperatur zwei Stunden lang gerührt, dann wird das Piperidin im Vakuum abdestilliert. Der Rückstand wird im Gemisch von 20 ml Äthanol und 10 ml wäßriger n Natriumhydroxydlösung aufgenommen und eine halbe Stunde lang am Rückfluss gekocht, danach im Vakuum eingedampft. Der Rückstand wird in 20 ml Wasser aufgenommen, die Kristalle werden abfiltriert, mit Wasser gewaschen und getrocknet. So erhält man 1,74 g (86 %) der Titelverbindung, die bei Mischen mit dem Produkt gemäss Beispiel 1 keine Schmelzpunktdepression ergibt.

Beispiel 3

Herstellung von 6-Amino-1,2-dihydro-1-hydroxy-2-imino--4-piperidino-pyrimidin

Zum Gemisch von 10 ml Chloroform und 2 ml Piperidin werden unter Rühren 0,3 g (0,88 mMol) 6-Amino-1,2-dihydro--1-acetoxy-2-imino-4-p-toluol-sulfonyloxy-pyrimidin gegeben. Das Gemisch wird unter Rühren eine halbe Stunde lang gekocht und im Vakuum eingedampft. Es werden 5 ml Äthanol und 1 ml wässrigen Natriumhydroxydlösung zugegeben. Dann lässt man das Gemisch bei Reumtemperatur eine Stunde lang stehen, danach wird es im Vakuum eingedampft. Der Rückstand wird mit 10 ml Wasser vermischt, die Kristalle werden abfiltriert, mit Wasser gewaschen und getrocknet.
So erhält man 0,14 g (75 %) der Titelverbindung, die bei Vermischen mit dem Produkt gemäss Beispiel 1 keine Schmelzpunktdepression ergibt.

Beispiel 4

Herstellung von 6-Amino-1,2-dihydro-1-hydroxy-2--imino-4-piperidino-pyrimidin

Zum Gemisch von 10 ml Chloroform und 2 ml Piperidin werden unter Rühren 0,49 g (2 mMol) 6-Acetamidc-1,2-dihydro--1-acetoxy-2-imino-4-chlorpyrimidin gegeben. Dann wird das Gemisch eine halbe Stunde lang am Rückfluss gekocht und im Vakuum eingedampft. Der Rückstand wird im Gemisch von 10 ml Äthanol und 3 ml wäßriger n Natriumhydroxydlösung aufgelöst, dann bei Raumtemperatur eine Stunde lang stehengelassen und erneut im Vakuum eingedampft. Der Rückstand wird in 10 ml Wasser aufgenommen, die Kristalle werden abfiltriert, mit Wasser gewaschen und getrocknet. So erhält man 0,34 g (80 %) der Titelverbindung, die bei Vermischen mit dem Produkt gemäss Beispiel 1 keine Schmelzpunktdepression ergibt.

Beispiel 5

Herstellung von 6-Amino-1,2-dihydro-1-hydroxy-2-imino--4-piperidino-pyrimidin

Zu 760 ml wasserfreiem Piperidin werden bei einer Temperatur von 0 - 5 °C unter Rühren 76 g (0,2 Mol) 6-Acetamido--1,2-dihydro-1-acetoxy-2-imino-4-p-toluol-sulfonyloxypyrimidin gegeben. Das Rühren wird bei dieser Temperatur fortgesetzt, nach zwei Stunden lässt man die Temperatur des Gemisches sich auf Raumtemperatur erhöhen, und es wird 24 Stunden lang gerührt. Das Piperidin wird im Vakuum abdestilliert, und zum Rückstand werden 500 ml Wasser gegeben. Das Gemisch wird bis zum nächsten Tag in den Kühlschrank gestellt. Der abgesonderte Stoff wird abfiltriert, mit Wasser gewaschen und gründlich abgesaugt. Der auf dem Filter verbliebene Stoff wird dreimal mit 50 ml Äther suspendiert, gewaschen und dann getrocknet. So erhält man 23,0 g (55 %) der Titelverbindung.

Zur Mutterlauge werden 75 ml 10 gew.-%-ige Natriumhydroxydlösung gegeben, dann wird eingedampft. Zum Rückstand gibt man 200 ml Wasser, und der pH der Lösung wird auf 7 eingestellt. Die Lösung wird bis zum nächsten Tag im Kühlschrank gelassen, dann wird der auskristtallisierte Stoff abfiltriert, mit Wasser gewaschen und getrocknet. Man erhält weitere 8,9 g (21 %) der Titelverbindung.

So erhält man insgesamt 31,9 g (76 %) der Titelverbindung, die bei Vermischen mit dem Produkt gemäss Beispiel 1 keine Schmelzpunktdepression ergibt.

## Beispiel 6

Herstellung von 6-Amino-1,2-dihydro-1-hydroxy-2--imino-4-piperidino-pyrimidin

a) Herstellung von 6-Acetamido-1,2-dihydro-1-hydroxy--2-imino-4-piperidino-pyrimidin

Zum Gemisch von 20 ml Chloroform und 5 ml Piperidin werden 1,01 g (5 mMol) 6-Amino-2-acetamido-4-chlorpyrimidin--1-oxyd gegeben. Das Gemisch wird unter Rühren eine halbe Stunde lang gekocht. Die erhaltene Lösung wird gekühlt und dreimal mit 10 ml n Salzsäure extrahiert, dann dreimal mit 10 ml Wasser gewaschen. Die Chloroform-Phase wird über ausgeglühtem Natriumsulfat getrocknet und im Vakuum eingedampft. Der Rückstand wird mit 50 ml Äther verrieben, die Kristalle werden abfiltriert, mit Äther gewaschen und getrocknet. So erhält man 0,86 g (69 %) der Titelverbindung.
Schmelzpunkt: 204 - 205 °C.
IR-Spektrum (KBr): 1670, 1600, 1570, 1500 $cm^{-1}$
UV-Spektrum (EtOH): 245, 325 nm
NMR-Spektrum ($CDCl_3$ + $CD_3OD$): 1,63 (m, 6H), 2,30 (s, 3H), 3,57 (m, 6H), 7,04 (s, 1H).

b) Herstellung von 6-Amino-1,2-dihydro-1-hydroxy-2--imino-4-piperidino-pyrimidin

In dem Gemisch von 10 ml Äthanol und 4 ml n Natriumhydroxylösung werden 0,5 g des gemäss Abschnitt a) hergestellten 6-Acetamido-1,2-dihydro-1-hydroxy-2-imino-4-piperidino--pyrimidins gelöst. Die Lösung wird 30 Minuten lang gekocht, dann im Vakuum eingedampft, und der Rückstand wird in 10 ml Wasser aufgenommen. Die Kristalle werden abfiltriert, mit Wasser gewaschen, dann getrocknet. So erhält man 0,35 g (85 %) der Titelverbindung, die bei Vermischen mit dem Produkt gemäss Beispiel 1 keine Schmelzpunktdepression ergibt.

Beispiel 7

Herstellung von 6-Amino-1,2-dihydro-1-hydroxy-2--imino-4-piperidino-pyrimidin

a) Herstellung von 6-Acetamido-1,2-dihydro-1-acetoxy--2-imino-4-piperidino-pyrimidin

In dem Gemisch von 20 ml Acetonitril und 0,5 ml Piperidin werden 0,5 g (0,0013 Mol) 6-Acetamido-1,2-dihydro--1-acetoxy-2-imino-4-(p-toluol-sulfonyloxy)-pyrimidin bei Raumtemperatur 3 Stunden lang gerührt. Dann wird das Gemisch bei verringertem Druck eingedampft, und zum Rückstand den 30 ml Äther gegeben. Der entstandene weisse kristalline Stoff wird abfiltriert, erst mit Äther, dann mit Wasser gewaschen und getrocknet.

So erhält man 0,24 g (64 %) der Titelverbindung.

Schmelzpunkt: 217 - 218 °C (zerfällt).

IR-Spektrum (KBr): 1710, 1680, 1630, 1570, 1530 $cm^{-1}$

UV-Spektrum (EtOH): 241, 293, 323 nm

NMR-Spektrum ($CDCl_3$ + TFA-d): 1,76 (m, 6H), 2,43 (s, 3H), 2,57 (s, 3H), 3,80 (m, 4H), 7,55 (s, 1H).

b) Herstellung von 6-Amino-1,2-dihydro-1-hydroxy-2--imino-4-piperidino-pyrimidin

In dem Gemisch von 20 ml Äthanol und 5 ml n Natriumhydroxydlösung werden 1,0 g (3,4 mMol) des gemäss Abschnitt a) hergestellten 6-Acetamido-1,2-dihydro-1-acetoxy-2-imino--4-piperidino-pyrimidins gelöst. Die Lösung wird 30 Minuten lang gekocht, dann im Vakuum eingedampft. Der Rückstand wird in 10 ml Wasser aufgenommen, die Kristalle werden abfiltriert, mit Wasser gewaschen und getrocknet.

So erhält man 0,54 g (76 %) der Titelverbindung, die bei Vermischen mit dem Produkt gemäss Beispiel 1 keine Schmelzpunktdepression ergibt.

Beispiel 8

Herstellung von 6-Amino-1,2-dihydro-1-hydroxy-2--imino-4-piperidino-pyrimidin

2,0 g (0,0093 Mol) 6-Amino-1,2-dihydro-1-propionyloxy-2-imino-4-chlorpyrimidin in 10 ml Wasser werden in Gegenwart von 5 ml Piperidin eine halbe Stunde lang gekocht, dann werden 10 ml 1 molare wäßrige Natriumhydroxydlösung und 5 ml Wasser zugegeben. Nach einer weiteren halben Stunde Kochen wird die Lösung abgekühlt. Die Kristalle werden nach einstündigem Stehen abfiltriert und mit Wasser gewaschen.

So erhält man 1,36 g (83 %) der Titelverbindung.

Beispiel 9

Herstellung von 6-Amino-2-imino-1-hydroxy-4-piperidino--1,2-dihydro-pyrimidin

In einem mit Rührer und Thermometer ausgestatteten Rundkolben werden unter Rühren und Kühlen mit Eiswasser 40,8 g (0,1 Mol) rohes 6-Acetamido-1-acetoxy-2-imino-4--mesitylen-sulfonyloxy-1,2-dihydro-pyrimidin mit 380 ml (327 g; 3,84 Mol) Piperidin umgesetzt. Das Rühren wird nach Erwärmen des Reaktionsgemisches auf Raumtemperatur fortgesetzt, bis chromatographisch nur noch die Produkte wahrgenommen werden können. Dies entspricht einer Zeit von etwa 24 Stunden. Dann wird das Piperidin in einem Bad mit einer Temperatur von 60 °C im Vakuum abdestilliert, und zum Rückstand werden 250 ml Wasser gegeben. Der abgesonderte Stoff wird nach dem Abkühlen abfiltriert und mit Wasser gewaschen, dann auf dem Filter getrocknet. Aus dem auf dem Filter verbliebenen festen Stoff wird das als Nebenprodukt enstandene Sulfonsäureamid mit wenig Toluol herausgewaschen. Durch das Trockendestillieren der Toluollösung gewinnt man 3,9 g (14,5 %) Mesitylen-sulfonyl-piperidid. Die auf dem Filter verbliebenen 13 g Stoff sind das gewünschte Produkt.

Die Mutterlauge wird weiter aufgearbeitet. Man setzt ihr 38 ml 10 gew.-%-ige wäßrige Natriumhydroxydlösung zu und destilliert im Vakuum, bis sie ölartig wird. So wird das noch vorhandene Piperidin entfernt. Dann werden 100 ml Wasser zugegeben, und der pH-Wert der Lösung wird mit 10 gew.-%-iger Salzsäure auf 7 eingestellt. Nach längerem Kühlen wird der abgesonderte Stoff abfiltriert und mit Wasser gewaschen. So erhält man weitere 5,3 g des Stoffes.
Insgesamt werden 17,9 g (85,5 %) 6-Amino-2-imino-1-hydroxy-4-piperidino-1,2-dihydro-pyrimidin hergestellt.
Schmelzpunkt: 240 - 260 °C (Zersetzung).
Die chromatographischen und spektroskopischen Eigenschaften des Produktes stimmen mit den in den vorhergehenden Beispielen angegebenen überein.

Patentansprüche

Patentansprüche

1.) Verfahren zur Herstellung von 6-(Amino)-1,2-di--(hydro)-1-(hydroxy)-2-(imino)-4-(piperidino)--pyrimidin der Formel

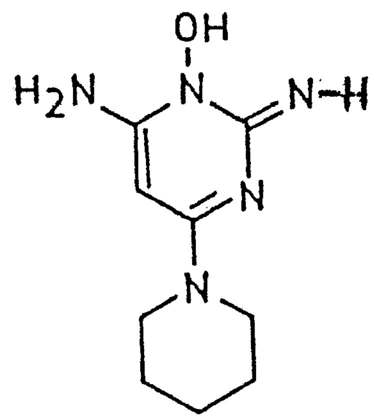

I ,

dadurch gekennzeichnet, daß man [eine] Acyl- und/ /oder Acyloxygruppe(n) aufweisenden 2-(Imino)-pyrimidinderivate der allgemeinen Formel

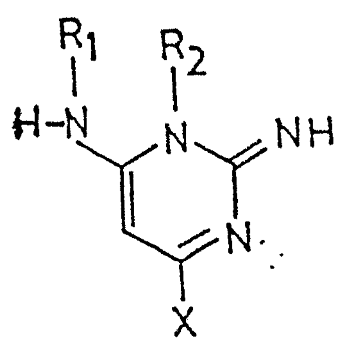

II ,

worin

$R_1$ für ein Wasserstoffatom oder einen Rest der allgemeinen Formel

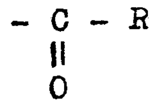 III ,

in welchletzterer

R einen Alkylrest mit 1 bis 6 Kohlenstoffatom(en) oder einen, gegebenenfalls durch ein Halogenatom substituierten, Arylrest bedeutet,

steht,

$R_2$ eine Hydroxygruppe oder einen Rest der allgemeinen Formel

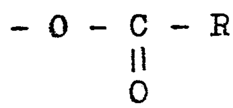 IV ,

in welchletzterer

R die obigen Bedeutungen hat,

darstellt und

X für ein Chlor- oder Bromatom oder einen, gegebenenfalls 1- oder mehrfach substituierten, Arylsulfonyloxyrest steht,

mit der weiteren Maßgabe, daß,

im Falle daß

$R_1$ für ein Wasserstoff atom steht,

$R_2$ von einer Hydroxygruppe verschieden ist,

mit Piperidin umsetzt und die erhaltenen [eine] Acyl- und/oder Acyloxygruppe(n) aufweisenden 2-(Imino)-4-(piperidino)-pyrimidinderivate der allgemeinen Formel

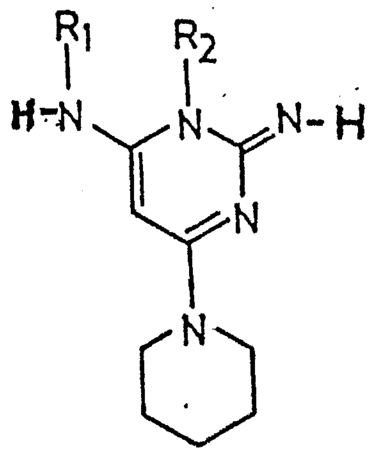

V ,

worin

$R_1$ und $R_2$ die obigen Bedeutungen haben,

gegebenenfalls nach ihrem Isolieren, hydrolysiert.

2.) Verfahren nach Anspruch 1, dadurch gekennzeichnet, daß man das Umsetzen der [eine] Acyl- und/oder Acyloxygruppe(n) aufweisenden 2-(Imino)-pyrimidinderivate der allgemeinen Formel II mit dem Piperidin bei Raumtemperatur und in Lösungsmitteln durchführt.

3.) Verfahren nach Anspruch 1 oder 2, dadurch gekennzeichnet, daß man als Lösungsmittel für die Umsetzung der [eine] Acyl- und/oder Acyloxygruppe(n) aufweisenden 2-(Imino)-pyrimidinderivate der allgemeinen Formel II mit dem Piperidin das Piperidin selbst verwendet.

4.) Verfahren nach Anspruch 1 oder 2, dadurch gekennzeichnet, daß man als Lösungsmittel für die Umsetzung der [eine] Acyl- und/oder Acyloxygruppe(n) aufweisenden 2-(Imino)-pyrimidinderivate der allgemeinen Formel II mit dem Piperidin protische und/oder dipolare und/oder apolare aprotische Lösungsmittel, insbesondere Äthanol, Acetonitril und/oder Chloroform, verwendet.

Zusammenfassung

Europäisches Patentamt

**EUROPÄISCHER RECHERCHENBERICHT**

0252515

Nummer der Anmeldung

EP 87 10 9986

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 4) |
|---|---|---|---|
| X | DE-A-2 943 161 (EGYT GYOGYSZERVEGYESZETI GYAR)<br>* Anspruch 1; Beispiel 4b * | 1,3 | C 07 D 239/50 |
| Y | * Seite 20, Zeilen 7-17 * & HH - B - 177 601 (Kat. D)<br>--- | 2,4 | |
| Y,D | DE-B-2 114 887 (THE UPJOHN CO.)<br>* Spalte 4, Zeilen 47-58; Spalte 5, Zeilen 17-33 *<br>--- | 2,4 | |
| A | DE-A-1 620 649 (THE UPJOHN CO.)<br>* Seite 10, Zeilen 9-14; Seite 12, Zeilen 10-17; Seite 48, Zeilen 11-17; Beispiele 9, 10 * & GB - A - 1 167 735 (Kat. D)<br>----- | 1 | |
| | | | **RECHERCHIERTE SACHGEBIETE (Int. Cl 4)** |
| | | | C 07 D 239/00 |

Der vorliegende Recherchenbericht wurde für alle Patentansprüche erstellt

| Recherchenort | Abschlußdatum der Recherche | Prüfer |
|---|---|---|
| BERLIN | 06-10-1987 | VAN AMSTERDAM L.J.P. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument